# EUROPÄISCHE PATENTSCHRIFT

(11) **EP 2 773 399 B1**
(45) Veröffentlichungstag und Bekanntmachung des Hinweises auf die Patenterteilung: **28.09.2016**
(21) Anmeldenummer: 12780397.1
(22) Anmeldetag: 15.10.2012
(51) Int. Cl.: A61M 5/24, A61M 5/32, A61M 5/31

(54) **VERABREICHUNGSVORRICHTUNG ZUM ABMISCHEN EINES WIRKSTOFFS MIT EINER LÖSUNGSFLÜSSIGKEIT**
DELIVERY DEVICE FOR MIXING AN ACTIVE INGREDIENT WITH A SOLUTION
DISPOSITIF DE DISTRIBUTION POUR MELANGER UN PRINCIPE ACTIF AVEC UNE SOLUTION

(30) Priorität: 03.11.2011 CH 17672011
(43) Veröffentlichungstag der Anmeldung: 10.09.2014
(73) Patentinhaber: TecPharma Licensing AG, 3401 Burgdorf (CH)
(72) Erfinder: KAUFMANN, Nadine, CH-3400 Burgdorf (CH); HIRSCHEL, Jürg, CH-3007 Bern (CH); TSCHlRREN, Markus, CH-3400 Burgdorf (CH)
(86) Internationale Anmeldenummer: PCT/CH2012/000235
(87) Internationale Veröffentlichungsnummer: WO 2013/063707

(56) Entgegenhaltungen:
- WO-A1-2007/131013
- GB-A- 2 319 184
- GB-A- 2 447 787

## Beschreibung

Die Erfindung betrifft eine Verabreichungsvorrichtung, insbesondere eine Verabreichungsvorrichtung mit einem Behälter und einer Abmischvorrichtung zum Abmischen eines Wirkstoffs mit einer Lösungsflüssigkeit in dem Behälter.

Aus der WO2007/131013A1 ist eine Abmischvorrichtung mit einer ZweikammerKarpule bekannt,wobei der Abmischvorgang mit Hilfe einer Feder durch eine Drehung und durch eine axiale Bewegung einer Kappe der Abmischvorrichtung ausgelöst wird.

Aus der WO2008/122132A1 ist eine Verabreichungsvorrichtung mit einer Abmischvorrichtung bekannt. Bei dieser Verabreichungsvorrichtung wird mittels einer relativen Drehung zwischen einer Zweikammerkarpule, welche einen Wirkstoff und eine Lösungsflüssigkeit aufgenommen hat, und einer Abmischvorrichtung der Wirkstoff mit der Lösungsflüssigkeit abgemischt.

Nachteilig bei der Verabreichungsvorrichtung gemäss WO2008/122132A1 ist, dass die Gefahr besteht, dass der Benutzer versucht den Wirkstoff mit der Lösungsflüssigkeit abzumischen, bevor die Nadel in die Zweikammerkarpule eingestochen worden ist und folglich ein wirkungsvolles Abmischen verhindert wird, da insbesondere ein beim Abmischen entstandenes Gas oder ein bereits in der Zweikammerkarpule enthaltenes Gas nicht entweichen kann. Des Weiteren besteht das Risiko, dass der Benutzer einen nur teilweise abgemischten fluiden Wirkstoff in die Haut injizieren kann.

Im Folgenden bedeutet die proximale Richtung bei einem Verabreichungsgerät mit einer Injektionsnadeleinheit die Richtung zum Verabreichungsgerät und die distale Richtung die Richtung zur Injektionsnadeleinheit.

Es ist eine Aufgabe der Erfindung eine Verabreichungsvorrichtung zur Verabreichung eines fluiden Wirkstoffs aus einem Behälter bereitzustellen, welche ein vorgängiges Einstechen der Injektionsnadel in den Behälter und ein Abmischen und/oder Primen des Wirkstoffs mit der Lösungsflüssigkeit für den Wirkstoff sicherstellt.

Die Aufgabe wird durch die Verabreichungsvorrichtung gemäss Anspruch 1 gelöst. Vorteilhafte Ausführungsformen einer solchen Verabreichungsvorrichtung gehen aus den abhängigen Ansprüchen hervor.

Die Erfindung geht von einer Verabreichungsvorrichtung zur Verabreichung eines fluiden Wirkstoffs aus einem Behälter mit wenigstens einer ersten Kammer mit einem Wirkstoff und einer zweiten Kammer mit einer Lösungsflüssigkeit für den Wirkstoff aus. Der Behälter kann eine Karpule, in welcher der Wirkstoff und die Lösungsflüssigkeit direkt aufgenommen sind, oder ein Karpulenhalter, der eine Karpule mit dem Wirkstoff und der Lösungsflüssigkeit umfasst, sein.

Der Behälter weist ferner zum Verschluss an einem Ende eine Membran auf. Vorzugsweise kann die erste Kammer des Behälters durch einen ersten Stopfen und die zweite Kammer durch den ersten und einen zweiten

Stopfen begrenzt sein. Die Kammern können via einem Bypass in einer Wand des Behälters verbindbar sein, um ein Abmischen des Wirkstoffs mit der Lösungsflüssigkeit zu ermöglichen.

Des Weiteren umfasst die Verabreichungsvorrichtung eine Abmischvorrichtung zum Abmischen des Wirkstoffs mit der Lösungsflüssigkeit für den Wirkstoff. Die Abmischvorrichtung kann gegen den zweiten Stopfen anschliessen. Durch eine Bewegung des Behälters relativ zu der Abmischvorrichtung kann der zweite Stopfen und der erste Stopfen aufgrund der Kraftübertragung der Lösungsflüssigkeit in der zweiten Kammer innerhalb des Behälters verschoben werden, bis der erste Stopfen in den Bereich des Bypasses gelangt ist. Bei einer weiteren Bewegung des Behälters relativ zu der Abmischvorrichtung wird der zweite Stopfen relativ zu dem Behälter bewegt, sodass die Lösungsflüssigkeit durch den Bypass in die erste Kammer gelangt und sich mit dem Wirkstoff vermischt. Die Abmischvorrichtung kann ein Antriebsglied, insbesondere eine Kolbenstange zu der Abmischung des Wirkstoffs mit der Lösungsflüssigkeit und zur Verabreichung des abgemischten Produkts und ein Gehäuse zur Aufnahme der Kolbenstange umfassen. Während dem Abmischvorgang ist der Behälter relativ zu der Kolbenstange und dem Gehäuse axial und vorzugsweise drehbar verschiebbar. Der Behälter kann vorzugsweise radial zwischen der Kolbenstange und dem Gehäuse in die Verabreichungsvorrichtung eingeschraubt werden. Die Kolbenstange kann als Zahnstange oder als Gewindestange ausgebildet sein und kann sowohl manuell betätigt wie auch durch einen elektrischen oder mechanischen Antrieb, insbesondere eine Feder angetrieben werden.

Die Abmischvorrichtung kann somit Teil einer Verabreichungsmechanik der Verabreichungsvorrichtung sein, wobei der Behälter von der Verabreichungsmechanik abgekoppelt werden kann, um einen neuen Behälter anzukoppeln, nachdem der alte entleerte Behälter entsorgt wurde. Die Verabreichungsmechanik kann ferner einen Auslöseknopf vorsehen, durch dessen Betätigung die Verabreichungsmechanik aktiviert wird, sodass durch eine an der Verabreichungsvorrichtung angebrachte Injektionsnadeleinheit ein abgemischter Wirkstoff aus dem Behälter in die Haut injiziert werden kann. Ferner kann die Verabreichungsvorrichtung eine weitere Sicherheitseinrichtung umfassen, die sicherstellt, dass eine Injektion nur nach einem vollständigen Abmischen und/oder Primen des Wirkstoffs mit der Lösungsflüssigkeit ausgeführt werden kann. Dazu kann die Verabreichungsmechanik eine Blockiereinrichtung aufweisen, welche die Betätigung des Auslöseknopfs verhindert, wenn sich die Blockiereinrichtung in der Blockierposition befindet. Die Blockiereinrichtung kann von der Blockierposition in eine Freigabeposition bewegt werden, in welcher der Auslöseknopf zur Verabreichung des abgemischten Wirkstoffs betätigt werden kann. Durch Drehung des Behälters relativ zu der Abmischvorrichtung kann die Blockiereinrichtung von der Blockierposition in die Freigabeposition bewegt werden. Somit kann mittels der Drehung des Behälters die Verabreichungsvorrichtung entsichert und die Verabreichungsmechanik mit dem Auslöseknopf ausgelöst werden. Eine solche Blockiereinrichtung ist in der WO2009/100550A1 beschrieben. Die WO2009/100550A1 wird hiermit durch Verweis vollständig in das vorliegende Dokument aufgenommen.

In einer Ausführungsform kann ferner die Kolbenstange derart ausgebildet sein, dass sie eine Halteeinrichtung umfasst, welche die Karpule in einem Karpulenhalter in einer definierten Position relativ zu dem Gehäuse hält. Diese Anordnung kann ein zu grosses Spiel zwischen den einzelnen Bauteilen der Verabreichungsvorrichtung verhindern und eine präzise Funktionsweise der Verabreichungsvorrichtung sicherstellen. Eine solche Anordnung ist in der WO2009/100549A1 beschrieben, wobei die WO2009/100549A1 hiermit durch Verweis vollständig in dieses Dokument aufgenommen wird.

Ferner umfasst die Verabreichungsvorrichtung eine Injektionsnadeleinheit, die einen von dem Behälter abgewandten distalen Nadelabschnitt zum Einstechen in die Haut und einen proximalen Nadelabschnitt aufweist, der in einer Ausgangsposition der Verabreichungsvorrichtung der Membran zugewandt gegenüber liegt und der in einer Abmischposition durch die Membran in den Behälter gestochen ist. Wenn der proximale Nadelabschnitt der Injektionsnadel durch die Membran in den Behälter eingestochen ist, kann der proximale Nadelabschnitt mit dem Behälter eine fluide Verbindung herstellen. Somit kann während dem Abmischen des Wirkstoffs mit der Lösungsflüssigkeit ein entstandenes Gas oder ein bereits in dem Behälter enthaltendes Gas während dem Abmischvorgang und/oder dem Primingvorgang durch die Injektionsnadel der Injektionsnadeleinheit entweichen.

Die Injektionsnadeleinheit kann als Sicherheitsnadeleinheit ausgebildet sein, die ein Schutzschild umfasst, welches während der Injektion in die Haut von einer distalen Position, in welcher das Schutzschild den distalen Nadelabschnitt zum Einstechen in die Haut umgibt, in eine proximale Position, in welcher der distale Nadelabschnitt frei liegt, bewegt werden kann. Nach der Injektion kann das Schutzschild erneut in eine distale Position gelangen, in welcher das Schutzschild unlösbar verriegelt wird, um eine erneute Injektion mit der benutzten Injektionsnadeleinheit zu verhindern. Eine solche Injektionsnadeleinheit ist in der WO2008/028394A1 beschrieben, wobei die WO2008/028304A1 hiermit durch Verweis vollständig in dieses Dokument aufgenommen wird.

Die Verabreichungsvorrichtung umfasst ferner eine Schutzhülse, welche um die Injektionsnadeleinheit koaxial angeordnet werden kann und welche in der Ausgangsposition relativ zu dem Behälter mit der von der Schutzhülse aufgenommenen Injektionsnadeleinheit in die proximale Richtung, insbesondere nur in die proximale Richtung bewegbar ist.

Nach einem weiteren Aspekt der vorliegenden Erfindung kann die Schutzhülse in der Ausgangsposition relativ zu dem Behälter drehbar sein.

Die Schutzhülse ist zudem in der Abmischposition drehfest mit dem Behälter verbindbar und kann relativ zu der Abmischvorrichtung gedreht werden, um den Wirkstoff mit der Lösungsflüssigkeit für den Wirkstoff abzumischen. Eine Drehmomentübertragung von der Schutzhülse auf den Behälter kann direkt über eine lösbare Verbindung zwischen der Schutzhülse und dem Behälter oder indirekt über ein Drehmomentübertragungsmittel, insbesondere über eine Hülse erfolgen, wobei das Drehmomentübertragungsmittel eine lösbare Verbindung mit der Schutzhülse und/oder dem Behälter eingehen kann. Bevor die drehfeste Verbindung zwischen der Schutzhülse und dem Behälter in der Abmischposition hergestellt werden kann, muss zumindest der proximale Nadelabschnitt der Injektionsnadeleinheit durch die Membran in den Behälter gestochen sein.

Vorzugsweise kann zwischen der Injektionsnadeleinheit und der Schutzhülse eine Hülse, insbesondere eine Führungshülse angeordnet sein. Die Führungshülse kann an einem proximalen Ende mit dem Behälter axial fest verbunden sein. Vorzugsweise kann die Führungshülse relativ zu dem Behälter drehbar sein. Die Verbindung zwischen der Führungshülse und dem Behälter kann durch einen Form- oder Kraftschluss hergestellt werden. Besonders bevorzugt kann in der Führungshülse eine Ausnehmung vorgesehen sein, die mit einem an dem Behälter angeordneten nach aussen abragenden Vorsprung, insbesondere einem nach aussen umlaufenden Keil derart zusammenwirkt, dass eine axial feste und vorzugsweise drehbare Verbindung zwischen der Führungshülse und dem Behälter herstellbar ist. Der Vorsprung des Behälters kann in die Ausnehmung der Führungshülse rasten. In einer alternativen Ausführungsform kann ein an der Führungshülse vorgesehener Vorsprung, insbesondere ein umlaufender Keil in eine an dem Behälter vorgesehene Ausnehmung einrasten.

Erfindungsgemäss vorteilhaft kann die Führungshülse in der Ausgangsposition oder in der Abmischposition relativ zu dem Behälter drehbar sein. Ferner kann die Schutzhülse in der Ausgangsposition oder in der Abmischposition relativ zu der Führungshülse drehfest sein.

Des Weiteren kann die Injektionsnadeleinheit vorzugsweise in der Führungshülse axial bewegbar aufgenommen sein. Die Injektionsnadeleinheit kann derart in der Führungshülse axial bewegbar aufgenommen sein, dass der proximale Nadelabschnitt der Injektionsnadeleinheit in der Ausgangsposition der Verabreichungsvorrichtung der Membran zugewandt gegenüber liegt und wobei der proximale Nadelabschnitt der Injektionsnadeleinheit in der Abmischposition durch die Membran in den Behälter gestochen ist.

Vorzugsweise kann die Schutzhülse ein spannbares Verriegelungselement aufweisen, welches in der Ausgangsposition derart mit der Führungshülse zusammenwirkt, dass die Schutzhülse mit der darin aufgenommenen Injektionsnadeleinheit relativ zu dem Behälter in die proximale Richtung, insbesondere nur in die proximale Richtung bewegbar ist. Das Verriegelungselement kann elastisch spannbar ausgebildet sein. Das Verriegelungselement kann laschenförmig sein, insbesondere als biegbare oder biegeelastische Lasche ausgebildet sein. Vorzugsweise kann das an der Schutzhülse einstückig angeformte Verriegelungselement oder das an der Schutzhülse angebrachte Verriegelungselement radial zu der Schutzhülse schwenkbar oder radial zu der Schutzhülse elastisch deformierbar sein. Vorzugsweise kann das Verriegelungselement in der Ausgangsposition mit der Führungshülse in Anschlagkontakt sein und eine relative Bewegung der Schutzhülse zu der Führungshülse oder zu der Injektionsnadeleinheit oder zum Behälter in die distale Richtung verhindern. Alternativ kann die Schutzhülse in der Ausgangsposition zuerst relativ zu der Führungshülse in die distale Richtung bewegt werden, bis eine weitere relative Bewegung in die distale Richtung durch einen Anschlagkontakt zwischen dem Verriegelungselements und der Führungshülse verhindert wird. Vorzugsweise kann die Führungshülse eine Ausnehmung aufweisen, die derart ausgebildet ist, dass eine Relativbewegung der Schutzhülse zu der Führungshülse in die proximale Richtung zugelassen wird und in die distale Richtung verhindert wird.

Eine Anschlagfläche des Verriegelungselements und eine Gegenanschlagfläche der Ausnehmung der Führungshülse bilden den Anschlagkontakt zwischen dem Verriegelungselement der Schutzhülse und der Ausnehmung der Führungshülse.

In der Ausgangsposition kann die Schutzhülse relativ zu der Führungshülse über eine kraft- oder formschlüssige Verbindung, insbesondere eine Nut-/Nockenverbindung axial festgehalten, insbesondere in die proximaler Richtung axial festgehalten werden. Der Benutzer kann eine Kraft in die proximale Richtung, welche die Haltekraft der Nut-/Nockenverbindung übersteigt, gegen die Schutzhülse aufbringen. Die Nut-/Nockenverbindung kann sich lösen und die mit der Injektionsnadeleinheit aufgenommene Schutzhülse kann relativ zu dem Behälter in die proximale Richtung bewegt werden.

Je nach relativer Anordnung der Nut-/Nockenverbindung zu dem Verriegelungselement der Schutzhülse und zu der Ausnehmung der Führungshülse kann in der Ausgangsposition eine relative Bewegung zwischen der Schutzhülse zu der Führungshülse in die distale Richtung zumindest teilweise möglich sein, bis das Verriegelungselement in Anschlagkontakt mit der Ausnehmung gelangt. Im Anschlagkontakt zwischen dem Verriegelungselement und der Ausnehmung kann die Schutzhülse relativ zu der Führungshülse nicht in die distale Richtung bewegt werden. Die Schutzhülse kann nicht von der Injektionsnadeleinheit abgezogen werden.

Ferner kann das Verriegelungselement der Schutzhülse in der Abmischposition derart mit der Führungshülse zusammenwirken, dass die Schutzhülse relativ zu dem Behälter in die distale Richtung bewegbar ist.

Die Führungshülse kann in einer Ausführungsform der Erfindung eine Kulissenführung aufweisen, wobei das Verriegelungselement der Schutzhülse entlang der Kulissenführung geführt werden kann. Die Kulissenführung kann an einem Ende der Führung eine Rampe und an dem anderen Ende der Führung eine steile Stirnkante aufweisen. In der Ausgangsposition kann das Verriegelungselement mit einer Anschlagfläche gegen die Stirnkante der Kulissenführung stossen. Das Verriegelungselement kann somit einen Anschlag und die Stirnkante der Kulissenführung einen Gegenanschlag bilden. Das Verriegelungselement der Schutzhülse kann über eine Einstechposition, wobei der proximale Nadelabschnitt der Injektionsnadel die Membran durchsticht, in eine Abmischposition gebracht werden. In der Abmischposition kann das Verriegelungselement über die rampenförmige Steigung gleiten. Durch eine relative Drehung der Schutzhülse zu der Führungshülse kann die Verabreichungsvorrichtung in die Abmischposition gelangen. Die Schutzhülse kann mit dem Verriegelungselement erst in die distale Richtung relativ zu der Führungshülse oder zu der Injektionsnadeleinheit oder zu dem Behälter bewegt werden, wenn eine relative Drehbewegung der Schutzhülse mit dem Verriegelungselement zu der Führungshülse stattgefunden hat. In der Abmischposition kann die Schutzhülse von der Injektionsnadeleinheit abgezogen werden.

In einer Ausführungsform der Erfindung kann das Verriegelungselement der Schutzhülse zur Erreichung der Abmischposition soweit relativ zu der Führungshülse in die proximale Richtung bewegt werden, bis einerseits der proximale Nadelabschnitt der Injektionsnadeleinheit die Membran durchstochen hat und bis andererseits das Verriegelungselement über das proximale Ende der Führungshülse geglitten ist und sich entspannt, insbesondere elastisch entspannt hat. Das Verriegelungselement kann nun in eine Ausnehmung des Behälters oder in eine von dem proximalen Ende der Führungshülse und einer Schulter des Behälters gebildeten Ausnehmung oder zwischen an dem Behälter angebrachte Vorsprünge, insbesondere Rippen ragen. Wenn nun die Schutzhülse relativ zu der Führungshülse oder zu der Injektionsnadeleinheit oder zum Behälter in die distale Richtung bewegt wird, wird das Verriegelungselement der Schutzhülse durch die Führungshülse vorgespannt, insbesondere elastisch vorgespannt und kann über die Führungshülse und deren Ausnehmung gleiten. Die Schutzhülse kann somit von der Injektionsnadeleinheit abgezogen werden.

Die drehfeste Verbindung zwischen der Schutzhülse und dem Behälter kann in der Abmischposition in einer Ausführungsform durch einen Eingriff zwischen dem Behälter, insbesondere dem an dem Behälter vorgesehenen Vorsprung und einem Schnapparm, der an der Führungshülse angeordnet ist, herstellbar sein. An der Führungshülse können auch mehrere Schnapparme und an dem Behälter mehrere Vorsprünge vorgesehen sein. Der an der Führungshülse angebrachte Schnapparm kann das Drehmomentübertragungsmittel bilden. Die Führungshülse kann mit der Schutzhülse eine drehfeste Verbindung, insbesondere eine in eine Drehrichtung drehfeste Verbindung eingehen. Die mit der Schutzhülse drehfest verbundene Führungshülse kann mittels dem an der Führungshülse vorgesehenem Schnapparm eine Drehbewegung der Schutzhülse auf den Behälter übertragen.

Wenn die Schutzhülse mit der darin aufgenommenen Injektionsnadeleinheit relativ zu dem Behälter in die proximale Richtung bewegt wird, kann die Schutzhülse über die Führungshülse gleiten und den Schnapparm radial nach innen vorspannen. Der Schnapparm kann in der Einstechposition und in der Abmischposition radial nach innen vorgespannt, insbesondere elastisch vorgespannt sein. Vorzugsweise kann der Schnapparm in der Abmischposition gegen den an dem Behälter angebrachten Vorsprung angreifen und den Eingriff zwischen dem Behälter und dem Schnapparm der Führungshülse bildet. Der Eingriff dient dazu, eine drehfeste Verbindung, insbesondere eine in eine Drehrichtung drehfeste Verbindung über den Schnapparm zwischen der Schutzhülse und dem Behälter herzustellen. Wenn der Benutzer die Schutzhülse ergreift und relativ zu der Abmischvorrichtung dreht, kann der Behälter über den Schnapparm der Führungshülse relativ zu der Abmischvorrichtung gedreht werden, um den Wirkstoff mit der Lösungsflüssigkeit für den Wirkstoff in dem Behälter abzumischen. Nachdem der Abmischvorgang beendet ist, kann bei einer weiteren relativen Drehung zwischen dem Behälter und der Abmischvorrichtung die Verabreichungsvorrichtung auch geprimt werden. Dabei wird ein in dem Behälter und/oder in der Injektionsnadel enthaltendes Gas ausgestossen, sodass die Verabreichungsvorrichtung zur Injektion in die Haut bereit ist.

Um in die Abmischposition zu gelangen, muss vorzugsweise die mit der Führungshülse zumindest in eine Drehrichtung drehfest verbundene Schutzhülse vorerst gedreht werden, bevor der an der Führungshülse angebrachte Schnapparm gegen den an dem Behälter angebrachten Vorsprung angreift und den Eingriff zwischen dem Behälter und dem Schnapparm bildet. In einer besonders bevorzugten Ausführungsform muss die Schutzhülse mit dem Verriegelungselement relativ zu der Führungshülse vorerst derart gedreht werden, sodass das Verriegelungselement entlang der Kulissenführung der Führungshülse geführt wird, bis eine in zumindest eine Drehrichtung drehfeste Verbindung zwischen dem Verriegelungselement und der Führungshülse gebildet ist. Bei einer weiteren Drehung der Schutzhülse nimmt das Verriegelungselement mittels eines Anschlagkontakts zwischen dem Verriegelungselement und der Kulissenführung die Führungshülse mit, bis der Schnapparm der Führungshülse an einen an dem Behälter vorgesehenen Vorsprung schlägt und den Behälter mitnimmt.

Der Benutzer kann somit durch eine relative Drehung der Schutzhülse zu der Abmischvorrichtung, den Behälter über die Führungshülse ebenfalls relativ zu der Abmischvorrichtung drehen und ein wirkungsvolles Abmischen des Wirkstoffs mit der Lösungsflüssigkeit bewirken. Insbesondere kann der Benutzer durch Drehung der Schutzhülse relativ zu der Abmischvorrichtung, den Behälter über die Führungshülse in die Verabreichungsvorrichtung radial zwischen das Gehäuse und das Antriebsglied, insbesondere der Kolbenstange einschrauben, wobei das Antriebsglied, insbesondere die Kolbenstange auf den zweiten Stopfen wirkt und einen Abmischvorgang und/oder Primingvorgang bewirkt.

In einer Ausführungsform kann die drehfeste Verbindung zwischen der Schutzhülse und dem Behälter in der Abmischposition durch einen Eingriff zwischen dem Behälter und der Schutzhülse oder dem Verriegelungselement der Schutzhülse hergestellt werden. Die Schutzhülse ist relativ zu der Führungshülse axial in die proximale Richtung bewegbar. In der Abmischposition kann das Verriegelungselement oder ein Teil der Schutzhülse in eine Ausnehmung des Behälters oder in eine von dem proximalen Ende der Führungshülse und einer Schulter des Behälters gebildeten Ausnehmung oder zwischen an dem Behälter angebrachte Vorsprünge, insbesondere Rippen, ragen und gegen einen an dem Behälter vorgesehenen Vorsprung, insbesondere eine Rippe oder gegen eine die Ausnehmung umfassende Anschlagfläche schlagen und somit den Eingriff zwischen dem Behälter und der Schutzhülse oder dem Verriegelungselement bilden. In der Abmischposition ist eine zumindest in eine Drehrichtung drehfeste Verbindung zwischen der Schutzhülse und dem Behälter hergestellt. Das Verriegelungselement oder das Teil der Schutzhülse kann sich im Wesentlichen entspannt oder elastisch entspannt in die Ausnehmung des Behälters oder in die von dem proximalen Ende der Führungshülse und einer Schulter des Behälters gebildeten Ausnehmung oder zwischen an dem Behälter angebrachte Vorsprünge, insbesondere Rippen erstrecken.

Vorzugsweise kann in der Abmischposition der Eingriff zwischen dem Behälter und der Schutzhülse oder dem Verriegelungselement derart gebildet sein, dass vorerst die Schutzhülse relativ zu dem Behälter gedreht werden muss, bevor das Verriegelungselement oder ein Teil der Schutzhülse in Eingriff mit dem Behälter gelangt.

Durch eine relative Drehung der Schutzhülse zu der Abmischvorrichtung kann der Behälter relativ zu der Abmischvorrichtung gedreht werden, insbesondere relativ zu der Verabreichungsvorrichtung radial zwischen einem Gehäuse und der Kolbenstange eingeschraubt werden. Der Abmischvorgang und/oder Primingvorgang kann somit wirksam bewerkstelligt werden.

Der Eingriff zwischen dem Behälter und dem Schnapparm der Schutzhülse wie auch der Eingriff zwischen dem Verriegelungselement oder dem Teil der Schutzhülse kann auch durch einen anderen form- und/oder kraftschlüssigen Eingriff gebildet sein. Der Eingriff kann eine Rippenverbindung, Nut-/Keilverbindung, Stift-/Bohrungsverbindung, Anschlagverbindung oder Schnappverbindung sein.

Vorzugsweise kann die Verabreichungsvorrichtung eine Anzeigevorrichtung aufweisen, die zumindest eine erste Anzeige zur Markierung einer Stellung des Behälters relativ zu der Abmischvorrichtung nach Beendigung des Abmischvorgangs und eine zweite Anzeige zur Markierung einer Stellung des Behälters relativ zu der Abmischvorrichtung nach Beendigung des Primingvorgangs umfasst. Dazu kann eine visuelle, akustische oder taktile Anzeige an der Verabreichungsvorrichtung, insbesondere an dem Behälter vorgesehen sein, welche die relative Position des Behälters zu der Abmischvorrichtung anzeigt. Ferner können auch Raststellungen zur Anzeige der einzelnen Position bei den einzelnen Schritten der Vorbereitung der Verabreichungsvorrichtung, wie dem Abmischen und Primen vorgesehen werden, welche durch eine Rückdrehsicherung gebildet sind, durch die es nicht mehr möglich ist, den Behälter entgegen seiner Einführrichtung aus dem Gehäuse zu entfernen.

Die Erfindung wird im Folgenden an mehreren Figuren beschrieben. Die hierbei offenbarten Merkmale bilden je einzeln und in Kombination die Erfindung vorteilhaft weiter. Es zeigen:
- Figur 1a: eine erste Ausführungsform einer erfindungsgemässen Verabreichungsvorrichtung in einer Ausgangsposition in Aussenansicht
- Figur 1b: die Verabreichungsvorrichtung der ersten Ausführungsform in der Ausgangsposition im Längsschnitt
- Figur 2: vergrösserte Detailansicht im Längsschnitt einer auf der Verabreichungsvorrichtung aufgesetzten Injektionsnadeleinheit aus Figur 1 b
- Figur 3: perspektivische Darstellung der Position eines Verriegelungselements einer Schutzhülse in einer Kulissenführung einer Führungshülse der Verabreichungsvorrichtung der ersten Ausführungsform in der Ausgangsposition
- Figur 4: vergrösserte perspektivische Detailansicht im Längsschnitt der auf der Verabreichungsvorrichtung aufgesetzten Injektionsnadeleinheit aus Figur 1 b
- Figur 5: vergrösserte perspektivische Detailansicht in Aussenansicht der auf der Verabreichungsvorrichtung aufgesetzten Injektionsnadeleinheit aus Figur 1b
- Figur 6a: die Verabreichungsvorrichtung der ersten Ausführungsform in einer Einstechposition in Aussenansicht
- Figur 6b: die Verabreichungsvorrichtung der ersten Ausführungsform in der Einstechposition im Längsschnitt
- Figur 7: vergrösserte Detailansicht im Längsschnitt der auf der Verabreichungsvorrichtung aufgesetzten Injektionsnadeleinheit aus Figur 6b
- Figur 8: perspektivische Darstellung der Position des Verriegelungselements der Schutzhülse in der Kulissenführung der Führungshülse der Verabreichungsvorrichtung der ersten Ausführungsform in der Einstechposition
- Figur 9: vergrösserte perspektivische Detailansicht im Längsschnitt der auf der Verabreichungsvorrichtung aufgesetzten Injektionsnadeleinheit aus Figur 6b
- Figur 10a: die Verabreichungsvorrichtung der ersten Ausführungsform in der Abmischposition in Aussenansicht
- Figur 10b: die Verabreichungsvorrichtung der ersten Ausführungsform in der Abmischposition im Längsschnitt
- Figur 11: perspektivische Darstellung der Position des Verriegelungselement der Schutzhülse in der Kulissenführung der Führungshülse der Verabreichungsvorrichtung der ersten Ausführungsform in der Abmischposition
- Figur 12a: die Verabreichungsvorrichtung der ersten Ausführungsform in der abgemischten und/oder geprimten Position in Aussenansicht, wobei die Schutzhülse von der Injektionsnadeleinheit abgezogen werden kann
- Figur 12b: die Verabreichungsvorrichtung der ersten Ausführungsform in der abgemischten und/oder geprimten Position im Längsschnitt, wobei die Schutzhülse von der Injektionsnadeleinheit abgezogen werden kann
- Figur 13: perspektivische Darstellung der Position des Verriegelungselements der Schutzhülse in der Kulissenführung der Führungshülse der Verabreichungsvorrichtung der ersten Ausführungsform beim Abziehen der Schutzhülse von der Injektionsnadeleinheit
- Figur 14a: die Verabreichungsvorrichtung der ersten Ausführungsform im ausgeschütteten Zustand in Aussenansicht
- Figur 14b: die Verabreichungsvorrichtung der ersten Ausführungsform im ausgeschütteten Zustand im Längsschnitt
- Figur 15a: eine zweite Ausführungsform einer erfindungsgemässen Verabreichungsvorrichtung in einer Ausgangsposition in Aussenansicht
- Figur 15b: die Verabreichungsvorrichtung der zweiten Ausführungsform in der Ausgangsposition im Längsschnitt
- Figur 16: vergrösserte Detailansicht im Längsschnitt einer auf der Verabreichungsvorrichtung aufgesetzten Injektionsnadeleinheit aus Figur 15b
- Figur 17: vergrösserte Detailansicht im Längsschnitt des Eingriffs zwischen einem Verriegelungselement einer Schutzhülse und einer Ausnehmung einer Führungshülse in der Ausgangsposition der auf der Verabreichungsvorrichtung aufgesetzten Injektionsnadeleinheit aus Figur 15b
- Figur 18a: die Verabreichungsvorrichtung der zweiten Ausführungsform in einer Abmischposition in Aussenansicht
- Figur 18b: die Verabreichungsvorrichtung der zweiten Ausführungsform in der Abmischposition im Längsschnitt
- Figur 19: vergrösserte Detailansicht im Längsschnitt der auf der Verabreichungsvorrichtung aufgesetzten Injektionsnadeleinheit aus Figur 18b
- Figur 20: vergrösserte Detailansicht im Längsschnitt des Eingriffs zwischen dem Verriegelungselement der Schutzhülse und dem Vorsprung des Karpulenhalters in der Abmischposition der auf der Verabreichungsvorrichtung aufgesetzten Injektionsnadeleinheit aus Figur 18b

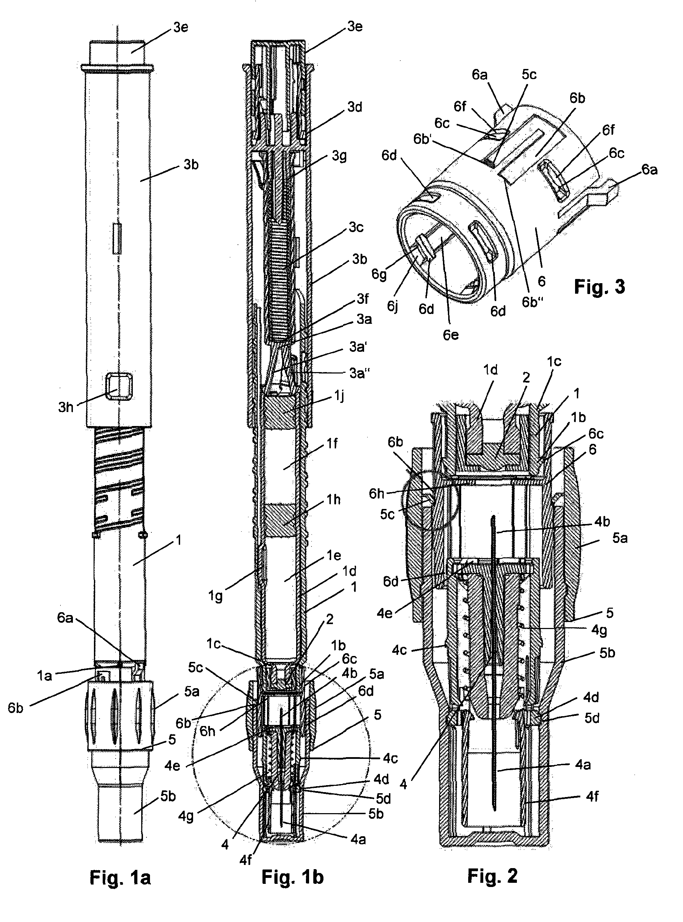

In den Figuren 1a und 1b ist eine erste Ausführungsform einer erfindungsgemässen Verabreichungsvorrichtung in einer Ausgangsposition in Aussenansicht und in Längsschnitt dargestellt. Die Verabreichungsvorrichtung umfasst einen zylinderförmigen Karpulenhalter (1), in dem eine Karpule (1d) gelagert ist, und eine Abmischvorrichtung zum Abmischen eines in einer ersten Kammer (1e) der Karpule (1d) aufgenommenen Wirkstoffs mit einer Lösungsflüssigkeit, die in einer zweiten Kammer (1f) der Karpule (1d) enthalten ist. Die Kammern (1e, 1f) der Karpule (1d) sind via einem Bypass (1g) in einer Wand der Karpule (1d) verbindbar. Die Karpule (1d) weist einen ersten Stopfen (1h) und einen zweiten Stopfen (1j) auf. Der zweite Stopfen (1j) schliesst die Karpule (1d) am proximalen Ende ab. Am distalen Ende weist die Karpule (1d) eine Verjüngung auf, deren Öffnung durch eine Membran (2) abgeschlossen ist. Eine Injektionsnadeleinheit (4) ist am distalen Ende der Verabreichungsvorrichtung auf die Verabreichungsvorrichtung aufgesetzt und über eine zylinderförmige Führungshülse (6) und den Karpulenhalter (1) mit der Karpule (1d) verbunden. Ein proximaler Nadelabschnitt (4b) der Injektionsnadeleinheit (4) liegt in der Ausgangsposition der Verabreichungsvorrichtung gegenüber der Membran (2) der Karpule (1d), wobei der proximale Nadelabschnitt (4b) am distalen Ende der Führungshülse (6) in die Führungshülse (6) ragt. Eine hülsenförmige Schutzhülse (5) umgibt die Injektionsnadeleinheit (4). Die Schutzhülse (5) umfasst einen Griffteil (5a) mit Längsrippen, welche dem Benutzer ein besseres Greifen der Schutzhülse (5) ermöglichen, und einen Schutzteil (5b), welches die Injektionsnadeleinheit (4) umgibt, sodass der Benutzer an einer Verletzung mit einer Injektionsnadel der Injektionsnadeleinheit (4), insbesondere einem distalen Nadelabschnitt (4a) geschützt wird. Der Griffteil (5a) und der Schutzteil (5b) der Schutzhülse sind axial fest und drehfest miteinander verbunden.

Die Injektionsnadeleinheit (4) ist als Sicherheitsnadeleinheit ausgebildet, wobei die Injektionsnadeleinheit (4) ein Schutzschild (4f) umfasst. Das Schutzschild (4f) ist derart an der Injektionsnadeleinheit (4) angeordnet, sodass das Schutzschild (4f) während der Injektion in die Haut von einer distalen Position, in welcher das Schutzschild (4f) den distalen Nadelabschnitt (4a) zum Einstechen in die Haut umgibt, in eine proximale Position, in welcher der distale Nadelabschnitt (4a) frei liegt, bewegbar ist. Nach der Injektion kann sich das Schutzschild (4f) aufgrund einer Beschlagung einer Federkraft einer Feder (4g) der Sicherheitsnadeleinheit (4) wieder in die distale Positon begeben, wobei das Schutzschild (4f) mittels einer Verriegelungseinrichtung unlösbar verriegelt wird, um eine erneute Injektion mit der benutzen Injektionsnadeleinheit (4) zu verhindern.

Eine Kolbenstange (3a) und ein Gehäuse (3b) bilden die Abmischvorrichtung. Der Karpulenhalter (1) ist radial zwischen der Kolbenstange (3a) und dem Gehäuse (3b) angeordnet. Der Karpulenhalter (1) weist ein Aussengewinde auf, welches in Gewindeeingriff mit einem Innengewinde des Gehäuses (3b) steht. Die Kolbenstange (3a) ist hülsenförmig ausgebildet. Die Kolbenstange (3a) umfasst vorzugsweise zwei Haltearme (3a', 3a"), welche die Karpule (1d) in dem Karpulenhalter (1) in einer definierten Position relativ zu dem Gehäuse (3b) halten. Im Innern der Kolbenstange (3a) ist vorzugsweise eine Feder (3c) zur automatischen Auslösung der Verabreichungsvorrichtung angeordnet, die zwischen einem distalen Anschlag am Hülsenboden (3f) der Kolbenstange (3a) und einem proximalen Anschlag an einem gehäusefesten Element (3g) eingespannt ist. Vorzugsweise kann die Verabreichungsvorrichtung einen Auslöseknopf (3e) und einen Blockierring (3d) aufweisen, wobei der Auslöseknopf (3e) und der Blockierring (3d) derart ausgebildet sind, dass der Blockierring (3d) eine Betätigung des Auslöseknopfs (3e) in der Ausgangsposition blockiert. Die Blockierung stellt sicher, dass die Verabreichungsvorrichtung erst nach dem vollständigen Abmischvorgang und/oder Primingvorgang durch Betätigung des Auslöseknopfs (3e) ausgelöst werden kann.

Alternativ kann die Verabreichungsvorrichtung durch eine manuelle Betätigung des Auslöseknopfs (3e) ausgelöst werden, wozu die Kolbenstange (3a) keine Feder (3c) umfasst. Des Weiteren kann alternativ die Verabreichungsvorrichtung keinen Blockierring (3d) und/oder die Kolbenstange (3a) keine Haltearme (3a', 3a") und/oder die Injektionsnadeleinheit (4) kein Schutzschild (4f) aufweisen.

In Figur 2 ist eine vergrösserte Detailansicht im Längsschnitt der auf der Verabreichungsvorrichtung aufgesetzten Injektionsnadeleinheit (4) in der Ausgangsposition dargestellt. Die Injektionsnadeleinheit (4) umfasst einen von dem Karpulenhalter (1) abgewandten distalen Nadelabschnitt (4a) zum Einstechen in die Haut und einen proximalen Nadelabschnitt (4b), der in der Ausgangsposition der Verabreichungsvorrichtung dem Karpulenhalter (1) zugewandt gegenüber liegt. Die Injektionsnadeleinheit (4) ist axial fest und vorzugsweise drehfest mit der Schutzhülse (5) verbunden. Dazu weist die Injektionsnadeleinheit (4) eine an der Mantelaussenfläche vorgesehene nach aussen ragende Nocke oder Ring (4d) auf, die oder der in eine an der Mantelinnenfläche vorgesehene Ausnehmung oder Ringnut (5d) der Schutzhülse (5) ragt. Der Karpulenhalter (1) umfasst an dessen distalen Ende eine stirnseitig vorgesehene Öffnung, welche in der axialen Flucht zur Membran (2) der Karpule (1d) angeordnet ist. Die Führungshülse (6) ist konzentrisch zwischen der Injektionsnadeleinheit (4) und der Schutzhülse (5) angeordnet, wobei die Führungshülse (6) mit dem proximalen Ende mit dem Karpulenhalter (1) axial fest und drehbar verbunden ist. Um die axial feste und drehbare Verbindung zu bilden, ist in der Führungshülse (6) eine Ausnehmung (6c) vorgesehen, in welche ein an dem Karpulenhalter (1) vorgesehener Vorsprung (1b), insbesondere ein in Umfangsrichtung erstreckender nach aussen ragender Keil (1b) einrasten kann. Der Keil (1b) kann derart eine steile und eine flache Flanke aufweisen, dass die flache Flanke der Führungshülse (6) bei der Montage auf den Karpulenhalter (1) relativ zu dem Karpulenhalter (1) über das rampenförmig ausgebildete proximale Ende des Karpulenhalters (1) geschoben werden kann, bis der Keil (1b) des Karpulenhalters (1) in die Ausnehmung (6c) der Führungshülse (6) gelangt. Die steile Flanke des Keils (1b) des Karpulenhalters (1) ist in Anschlagkontakt mit einer Stirnseite der Führungshülse (6), welche durch die Ausnehmung (6c) der Führungshülse (6) gebildet ist. Das distale Ende des Karpulenhalters (1) kann gegen eine proximale Seite einer Gegenanschlagebene (6h) der Führungshülse (6) anschlagen.

Die Schutzhülse (5) ist in der Ausgangsposition drehfest mit der Führungshülse (6) verbunden, wobei die Schutzhülse (5) relativ zu der Führungshülse (6) in Gleitkontakt axial bewegbar ist. Die drehfeste Verbindung zwischen der Schutzhülse (5) und der Führungshülse (6) kann durch eine Anschlagverbindung hergestellt werden.

Die Injektionsnadeleinheit (4) ist in der Führungshülse (6) axial bewegbar aufgenommen. Die Injektionsnadeleinheit (4) ist derart in der Führungshülse (6) axial bewegbar, dass der proximale Nadelabschnitt (4b) der Injektionsnadeleinheit (4) in der Ausgangsposition der Verabreichungsvorrichtung der Membran (2) zugewandt gegenüber liegt und in der Abmischposition durch die Membran (2) in den Behälter (1) gestochen ist. Die Injektionsnadeleinheit (4) ist über einen Gleitkontakt innerhalb der Führungshülse (6) und relativ zu der Führungshülse (6) geführt. Die Injektionsnadeleinheit (4) ist derart in der Führungshülse (6) axial bewegbar gelagert, dass der proximale Nadelabschnitt (4b) in der Abmischposition durch die Öffnung des Karpulenhalters (1) in die Membran (2) der Karpule (1d) durchdringen kann, um eine fluide Verbindung zwischen der Injektionsnadel der Injektionsnadeleinheit (4) und der Karpule (1d) zu bilden.

Die mit der Injektionsnadeleinheit (4) axial fest aufgenommene Schutzhülse (5) ist in der Ausgangsposition relativ zu dem mit der Karpule (1d) aufgenommenen Karpulenhalter (1) nur in die proximale Richtung bewegbar. Dazu weist die Schutzhülse (5) ein spannbares, insbesondere elastisch spannbares Verriegelungselement (5c) auf, welches in der Ausgangsposition derart mit einer in der Führungshülse (6) vorgesehenen Kulissenführung (6b) zusammenwirkt, dass die Schutzhülse (5) mit der darin aufgenommenen Injektionsnadeleinheit (4) relativ zu dem Karpulenhalter (1) nur in die proximale Richtung bewegbar ist.

In der Ausgangsposition wird die Schutzhülse (5) relativ zu der Führungshülse (6) über eine Nut-/Nockenverbindung axial festgehalten, insbesondere in die proximale Richtung axial festgehalten. Der Benutzer kann eine Kraft in die proximale Richtung, welche die Haltekraft der Nut-/Nockenverbindung übersteigt, gegen die Schutzhülse (5) aufbringen, Die Nut-/Nockenverbindung löst sich und die mit der Injektionsnadeleinheit (4) aufgenommene Schutzhülse (5) kann relativ zu dem Karpulenhalter (1) in die proximale Richtung bewegt werden. Eine relative Bewegung zwischen der Schutzhülse (5) und der Führungshülse (6) in die distale Richtung wird durch den Anschlagkontakt zwischen dem Verriegelungselement (5c) der Schutzhülse (5) und der Kulissenführung (6b) der Führungshülse (6) verhindert.

Die Figur 3 zeigt die Führungshülse (6) in Zusammenwirkung mit dem Verriegelungselement (5c) der Schutzhülse (5) in der Ausgangsposition. Das Verriegelungselement (5c) der Schutzhülse (5) ist im Eingriff mit der Kulissenführung (6b) der Führungshülse (6). Die Kulissenführung (6b) ist U-förmig ausgebildet und weist an einem Ende eine steile Stirnkante (6b') auf, wobei das andere Ende (6b") rampenförmig ausgebildet ist. Das Verriegelungselement (5c) ist als biegbare, insbesondere biegeelastische Lasche geformt. Eine Anschlagfläche (5c') des Verriegelungselements (5c) kann in der Ausgangsposition mit der steilen Stirnkante (6b') der Kulissenführung (6b) der Führungshülse (6) einen Anschlagkontakt bilden, um zu verhindern, dass die Schutzhülse (5) in der Ausgangsposition mit der darin aufgenommenen Injektionsnadeleinheit (4) in die distale Richtung bewegbar ist, insbesondere von der Injektionsnadeleinheit (4) abgezogen werden kann.

An dem proximalen Ende der Führungshülse (6) ist zumindest ein nach innen spannbarer Schnapparm (6a) vorgesehen. Der Schnapparm (6a) ist hackenförmig ausgebildet und ragt von der äusseren Manteloberfläche der Führungshülse (6) nach aussen ab.

Die Ausnehmung (6c) der Führungshülse (6), welche mit dem Keil (1b) des Karpulenhalters (1) zusammenwirken kann, um eine axial feste und drehbare Verbindung zwischen der Führungshülse (6) und dem Karpulenhalter (1) zu bilden, ist schlitzförmig ausgebildet. Die Mantelinnenfläche der Führungshülse (6) weist einen nach innen ragenden Längssteg (6e) auf, wobei die schlitzförmige Ausnehmung (6c) im Bereich des Längsstegs (6e) angeordnet ist. An dem Karpulenhalter (1) ist der in Umfangsrichtung erstreckender nach aussen ragender Keil (1b) angebracht. Um eine axial feste und drehbare Verbindung zwischen der Führungshülse (6) und dem Karpulenhalter (1) sicherzustellen, kann einerseits die steile Seite des Keils (1b) in Richtung der Ausnehmung (6c) ragen und gegen die durch den Längssteg (6e) und der Ausnehmung (6c) gebildete Anschlagfläche (6f) anschlagen und andererseits kann das distale Ende des Karpulenhalters (1) gegen die proximale Seite der Gegenanschlagebene (6h) der Führungshülse (6) stossen.

Die Führungshülse (6) weist eine zusätzliche Ausnehmung (6d) in axialer Flucht zu der proximalen Ausnehmung (6c), welche mit dem umlaufenden Keil (1b) des Karpulenhalters (1) zur axialen und drehbaren Sicherung der Führungshülse (6) mit dem Karpulenhalter (1) dient, auf. Die beiden Ausnehmungen (6c, 6d) sind im Wesentlichen gleich ausgebildet. Die an dem distalen Ende der Führungshülse (6) vorgesehene Ausnehmung (6d) ermöglicht eine axial feste Verbindung der Injektionsnadeleinheit (4) mit der Führungshülse (6), wenn die Injektionsnadeleinheit in der Abmischposition ist, wobei ein an der Injektionsnadeleinheit (4) nach aussen ragender Vorsprung (4c) in die distale Ausnehmung (6d) der Führungshülse (6) gelangt.

Der an der Mantelinnenseite der Führungshülse (6) angebrachte Längssteg (6e) ist in Gleitkontakt mit der Injektionsnadeleinheit (4), wobei die Injektionsnadeleinheit (4) relativ zu der Führungshülse (6) bewegbar ist. Der Gleitkontakt zwischen der Führungshülse (6) und der Injektionsnadeleinheit (4) dient der Längsführung der von der Schutzhülse (5) aufgenommenen Injektionsnadeleinheit (4) von der Ausgangsposition, in welcher der proximale Nadelabschnitt (4b) der Injektionsnadeleinheit (4) der Membran (2) der Karpule (1d) zugewandt gegenüber liegt, in die Abmischposition, in welcher der proximale Nadelabschnitt (4b) durch die Membran (2) in die Karpule (1d) gestochen ist.

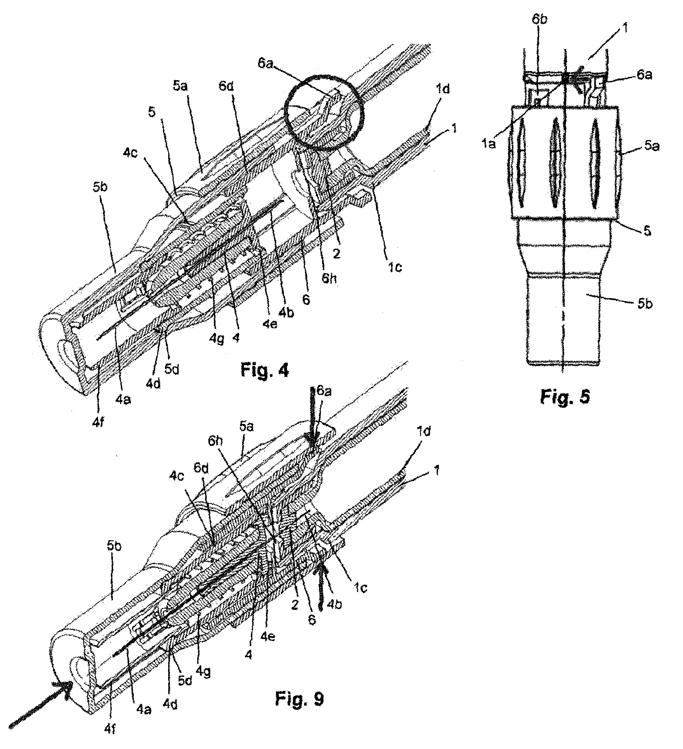

In Figur 4 ist eine vergrösserte perspektivische Detailansicht im Längsschnitt der auf der Verabreichungsvorrichtung aufgesetzten Injektionsnadel in der Ausgangsposition dargestellt. Der elastisch spannbare Schnapparm (6a) der Führungshülse (6) ist radial zwischen dem Karpulenhalter (1) und der Schutzhülse (5), insbesondere dem Griffteil (5a) der Schutzhülse entspannt (5) angeordnet und ragt über das proximale Ende des Griffteils (5a) der Schutzhülse (5) in Längsrichtung der Verabreichungsvorrichtung hervor. Wie in Figur 5 gezeigt, ist ein an dem Karpulenhalter (1) vorgesehener Vorsprung, insbesondere eine Nocke (1a) in Umlaufsrichtung versetzt zu dem Schnapparm (6a) der Führungshülse (6) vorgesehen. Die Schutzhülse (5) kann relativ zu der Verabreichungsvorrichtung, insbesondere der Abmischvorrichtung gedreht werden, wobei die mit der Schutzhülse (5) drehfest verbundene Führungshülse (6) in der Ausgangsposition mit gedreht wird. Das Verriegelungselement (5c) der Schutzhülse (5) schlägt an eine Flanke der Kulissenführung (6b) der Führungshülse (6) an und bildet die drehfeste Verbindung zwischen der Schutzhülse (5) und der Führungshülse (6). Der entspannte Schnapparm (6a) der Führungshülse (6) gelangt bei einer Drehung der Schutzhülse nicht in Anschlagkontakt mit der Nocke (1a) des Karpulenhalters (1), da der entspannte Schnapparm (6a) radial von der Nocke (1a) beabstandet ist. Der Karpulenhalter (1) kann folglich nicht über den Anschlagkontakt zwischen der Nocke (1a) des Karpulenhalters (1) und dem Schnapparm (6a) der Führungshülse (6) in das Gehäuse (3b) der Verabreichungsvorrichtung geschraubt werden, um in der Karpule (1d) den Wirkstoffs mit der Lösungsflüssigkeit abzumischen.

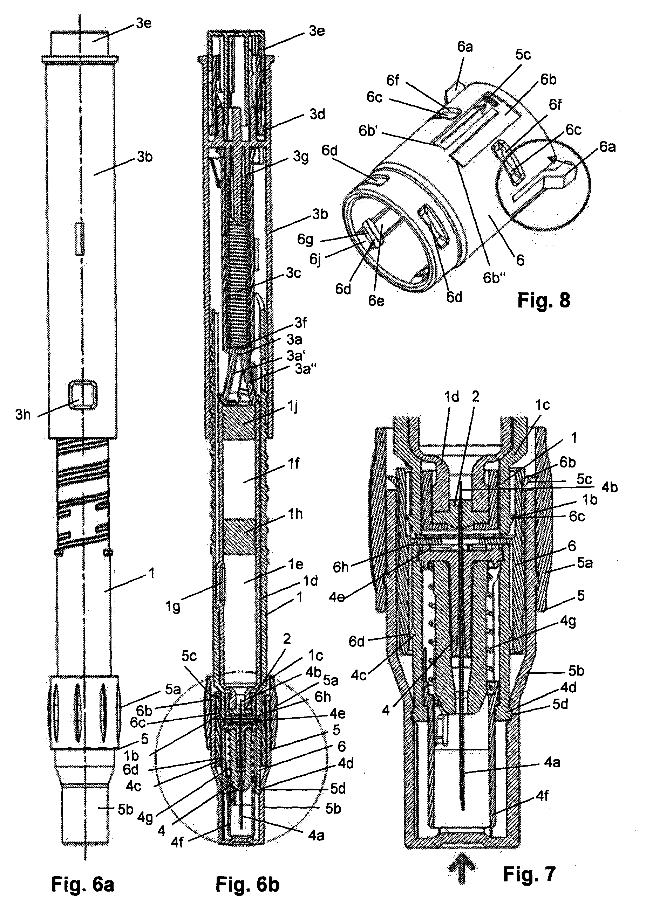

In den Figuren 6a und 6b ist die Verabreichungsvorrichtung der ersten Ausführungsform in einer Einstechposition in Aussenansicht und in Längsschnitt dargestellt, wobei Figur 7 eine vergrösserte Detailansicht im Längsschnitt der auf der Verabreichungsvorrichtung aufgesetzten Injektionsnadeleinheit (4) aus Figur 6b zeigt. Die mit der Injektionsnadeleinheit (4) aufgenommenen Schutzhülse (5) kann aufgrund des Anschlagkontakts zwischen dem Verriegelungselement (5c) der Schutzhülse (5) und der steilen Stirnkante (6b') der Kulissenführung (6b) der Führungshülse (6) in der Ausgangsposition nur in die proximale Richtung bewegt werden. Dazu drückt der Benutzer die Schutzhülse (5) in die proximale Richtung relativ zu der Abmischvorrichtung, um in die Einstechposition zu gelangen. Dabei bewegt sich die Schutzhülse (5) mit der axial fest verbundenen Injektionsnadeleinheit (4) in Gleitkontakt relativ zu der Führungshülse (6) bis die Injektionsnadeleinheit (4) mit einer Anschlagnocke oder einem Anschlagring (4e) gegen eine distale Seite der Gegenanschlagebene (6h) der Führungshülse (6), welche auf dem distalen Ende des Karpulenhalters (1) aufliegt, stösst. Wenn die Injektionsnadeleinheit (4) auf der distalen Seite der Gegenanschlagebene (6h) der Führungshülse (6) aufliegt, durchdringt der proximale Nadelabschnitt (4b) der Injektionsnadeleinheit (4) die Öffnung des Karpulenhalters (1) wie auch die Membran (2) der Karpule (1d), wobei eine fluide Verbindung zwischen der Injektionsnadel der Injektionsnadeleinheit (4) und der Karpule (1d) vorliegt.

Der an der Injektionsnadeleinheit (4) nach aussen ragende Vorsprung (4c) weist in die distale Richtung eine steile Flanke und in proximale eine flache Flanke auf. Der Vorsprung (4c) kann als an der Mantelaussenfläche der Injektionasnadeleinheit (4) angeordneter umlaufender Keil ausgebildet sein. Die flache Flanke des Vorsprungs (4c) gleitet bei der Bewegung in die Abmischposition über eine Rampe (6j) der Führungshülse (6) bis der Vorsprung (4c) in die distale Ausnehmung (6c) der Führungshülse (6) gelangt. Die Injektionsnadeleinheit (4) ist axial mit der Führungshülse (6) verbunden, da die steile Flanke des Vorsprungs (4c) gegen eine Anschlagfläche (6g) der Ausnehmung (6c) stösst und die Anschlagnocke oder der Anschlagring (4e) der Injektionsnadeleinheit (4) gegen die distale Seite der Gegenanschlagebene (6h) der Führungshülse (6) anschlägt.

Figur 8 zeigt die Position des Verriegelungselements (5c) der Schutzhülse (5) in der Kulissenführung (6b) der Führungshülse (6) in der Einstechposition. Das Verriegelungselement (5c) ist entlang der Kulissenführung (6b) in die proximale Position gelangt. Falls der Benutzer in dieser Position, ohne ein Abmischvorgang durchzuführen, die Schutzhülse (5) von der Injektionsnadeleinheit (4) abziehen möchte, bewegt sich das Verriegelungselement (5c) der Schutzhülse in die distale Richtung relativ zu der Führungshülse (6) bis das Verriegelungselement (5c) an die steile Stirnkante (6b') der Führungshülse (6) anschlägt. Die Schutzhülse (5) kann somit nicht ohne Abmischvorgang von der Injektionsnadeleinheit (4) abgezogen werden.

Aufgrund der axialen Verschiebung der mit der Injektionsnadeleinheit (4) axial fest verbundenen Schutzhülse (5) in die proximale Richtung relativ zu der Führungshülse (6) wird der an der Führungshülse (6) vorgesehene Schnapparm (6a) radial nach innen gespannt, wie in Figur 9 dargestellt ist. Bei der axialen Bewegung gleitet die Mantelinnenfläche der Schutzhülse (5) über den Schnapparm (6a) und drückt den Schnapparm (6a) radial nach innen.

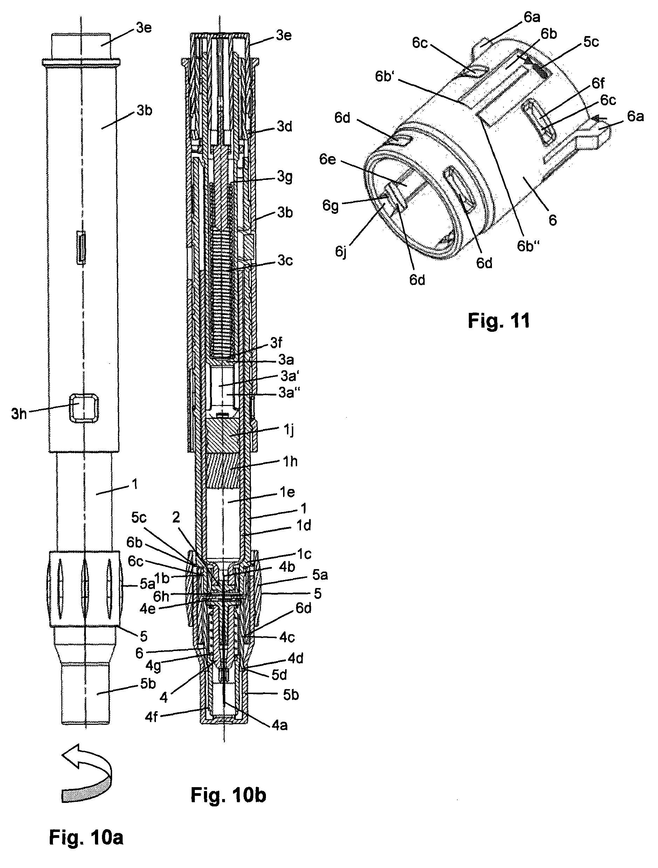

In den Figuren 10a und 10b ist die Verabreichungsvorrichtung der ersten Ausführungsform in einer Abmischposition in Aussenansicht und im Längsschnitt dargestellt. Zum Abmischen wird die Schutzhülse (5) relativ zu der Abmischvorrichtung der Verabreichungsvorrichtung gedreht. Dazu greift der Benutzer den Griffteil (5a) der Schutzhülse (5) und dreht die Schutzhülse (5) relativ zu dem Gehäuse (3b). Wie in Figur 11 dargestellt, dreht dabei die Schutzhülse (5) relativ zu der Führungshülse (6), bis das Verriegelungselement (5c) der Schutzhülse (5) in der Kulissenführung (6b) in Anschlagkontakt mit einer Flanke der Kulissenführung (6b) der Führungshülse (6) gelangt. Bei einer weiteren Drehung der Schutzhülse (5) nimmt das Verriegelungselement (5c) die Führungshülse (6) mit, bis der Schnapparm (6a) der Führungshülse (6) gegen die Nocke (1a) des Karpulenhalters (1) anstösst. Die Schutzhülse (5) ist über die Führungshülse (6) in zumindest eine Drehrichtung mit dem Karpulenhalter (1) drehfest verbunden, wobei das Verriegelungselement (5c) der Schutzhülse (5) in Anschlagkontakt mit der Flanke der Kulissenführung (6b) der Führungshülse (6) und der Schnapparm (6a) der Führungshülse (6) in Anschlagkontakt mit der Nocke (1a) des Karpulenhalters (1) ist. Aufgrund der drehfesten Verbindung zwischen der Schutzhülse und dem Karpulenhalter (1) kann durch eine Drehung der Schutzhülse (5) relativ zu der Abmischvorrichtung, nämlich dem Gehäuse (3b) und der Kolbenstange (3a), der Wirkstoff mit der Lösungsflüssigkeit für den Wirkstoff abgemischt werden. Der Karpulenhalter (1) wird zwischen das Gehäuse (3b) und die Kolbenstange (3a) eingeschraubt, wobei die Kolbenstange (3a) mittels den zwei Haltearmen (3a', 3a") an den zweiten Stopfen (1j) der Karpule (1d) stösst. Zum Einschrauben des Karpulenhalters (1) ist an der Innenseite des Gehäuses (3b) ein Innengewinde und an der Aussenseite des Karpulenhalters (1) ein Aussengewinde vorgesehen. Aufgrund der Kraftübertragung der Lösungsflüssigkeit zwischen dem zweiten und dem ersten Stopfen (1j, 1h) werden die beiden Stopfen (1h, 1j) in die distale Richtung innerhalb und relativ zu der Karpule (1d) verschoben, bis der erste Stopfen (1h) an den Bypass (1g) zu liegen kommt, durch den die Lösungsflüssigkeit in die erste Kammer (1e) fliessen kann und der zweite Stopfen (1j) an den ersten Stopfen (1h) zu liegen kommt. In der abgemischten Position ist der Wirkstoff der ersten Kammer (1e) mit der Lösungsflüssigkeit der zweiten Kammer (1f) abgemischt. Die Beendigung des Abmischens kann durch ein taktiles, akustisches und/oder visuelles Signal einer Anzeigevorrichtung (3h) angezeigt werden. Bei geringfügigem weiterem Eindrehen des Karpulenhalters (1) kann ein Primingvorgang durchgeführt werden, wobei dieser Vorgang zu einem weiteren Vorschub der beiden Stopfen (1h, 1j) führt, sodass ein in der Karpule (1d) und/oder in der Injektionsnadel enthaltenes Gas aus der Injektionsnadel entweichen kann, bis eine geringe Menge abgemischter Wirkstoff aus der Injektionsnadel der Injektionsnadeleinheit (4) austritt. Der Abschluss des Primingvorgangs kann durch ein taktiles, akustisches und/oder visuelles Signal der Anzeigevorrichtung (3h) angezeigt werden. Bei der letzten Einschraubbewegung des Karpulenhalters (1) in das Gehäuse (3b) kann der Blockierring (3d) von der Blockierposition in eine Freigabeposition bewegt werden. Durch eine Drehung kann ein Anschlag des Karpulenhalters (1) den Blockierring (3d) mitnehmen, sodass dieser relativ zum Gehäuse (3b) und zum Auslöseknopf (3e) gedreht wird. Durch diese Drehbewegung wird der Blockierring (3d) von der Blockierposition in die Freigabeposition bewegt. In der Freigabeposition kann der Auslöseknopf (3e) betätigt werden, indem der Auslöseknopf (3e) relativ zu dem Gehäuse (3b) entlang der Längsachse in das Gehäuse (3b) eingedrückt werden kann.

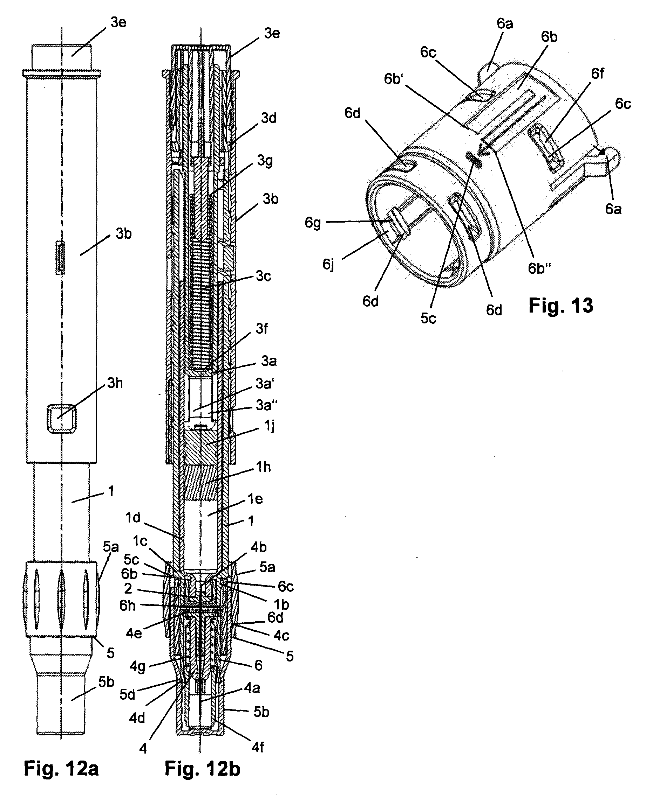

Die Figuren 12a und 12b zeigen die Verabreichungsvorrichtung der ersten Ausführungsform in der abgemischten und/oder geprimten Position in Aussenansicht und in Längsschnitt, wobei die Schutzhülse (5) von der Injektionsnadeleinheit (4) abgezogen werden kann. In der Figur 13 ist die Position des Verriegelungselements (5c) der Schutzhülse (5) in der Kulissenführung (6b) der Führungshülse (6) der Verabreichungsvorrichtung beim Abziehen der Schutzhülse (5) von der Injektionsnadeleinheit (4) gezeigt. Das Verriegelungselement (5c) ist nach einer relativen Drehung der Schutzhülse (5) zu der Führungshülse (6) in Anschlagkontakt mit der Flanke der Kulissenführung (6b). Wenn die Schutzhülse (5) relativ zu der Führungshülse (6) in die distale Richtung bewegt wird, gleitet das Verriegelungselement (5c) der Schutzhülse (5) entlang der Kulissenführung (6b) über die Rampe (6b") der Führungshülse (6). Die Schutzhülse (5) kann von der Injektionsnadeleinheit (4) abgezogen werden.

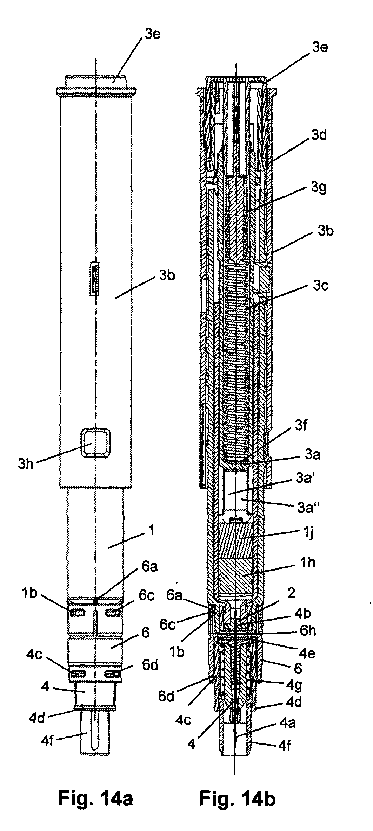

In den Figuren 14a und 14b ist die Verabreichungsvorrichtung der ersten Ausführungsform im ausgeschütteten Zustand in Aussensicht und im Längsschnitt dargestellt. Zum Auslösen der Verabreichungsvorrichtung kann der Auslöseknopf (3e) relativ zum Gehäuse (3b) entlang der Längsachse in das Gehäuse (3b) eingedrückt werden. Der Auslöseknopf (3e) ist derart ausgebildet, dass der Auslöseknopf (3e) bei Vorschub des Auslöseknopfs (3e) eine Sicherung der Kolbenstange (3a) an einem Gehäuseelement löst. In dem gelösten Zustand beginnt die Federkraft der Feder (3c) zu wirken und drückt gegen die Kolbenstange (3a). Die Kolbenstange (3a) wird durch die Kraft der Feder (3c) relativ zur Karpule (1d) verschoben, wobei die Haltearme (3a', 3a") der Kolbenstange (3a) die Stopfen (1j, 1h) innerhalb der Karpule (1d) antreiben, sodass der abgemischte Wirkstoff aus der ersten Kammer (1e) ausgeschüttet wird. Die Feder (3c) schiebt die Kolbenstange (3a) so lange in die Karpule (1d), bis ein Vorsprung, welcher an der Kolbenstange (3a) vorgesehen ist, an einer Kante des Gehäuses (3b) anschlägt. Sobald der Vorsprung am Gehäuse (3b) anschlägt, ist die Ausschüttung des abgemischten Wirkstoffs beendet. Ein taktiles, akustisches und/oder visuelles Signal der Anzeigevorrichtung (3h) kann anzeigen, dass die Ausschüttung beendet ist.

Nach der Injektion in die Haut kann die Schutzhülse (5) wieder auf die Injektionsnadeleinheit (4) aufgesetzt werden. Die Schutzhülse (5) kann relativ zu der Führungshülse (6) in die proximale Richtung bewegt werden, bis die Nut-/Nockenverbindung zwischen der Schutzhülse (5) und der Führungshülse (6) wieder hergestellt worden ist. Der mit der Karpule aufgenommene Karpulenhalter kann mit der Injektionsnadeleinheit von der Verabreichungsvorrichtung abgekoppelt werden, um einen Karpulenhalter mit einer neuen Karpule anzukoppeln.

Alternativ kann nach der Injektion die benutzte Verabreichungsvorrichtung entsorgt werden.

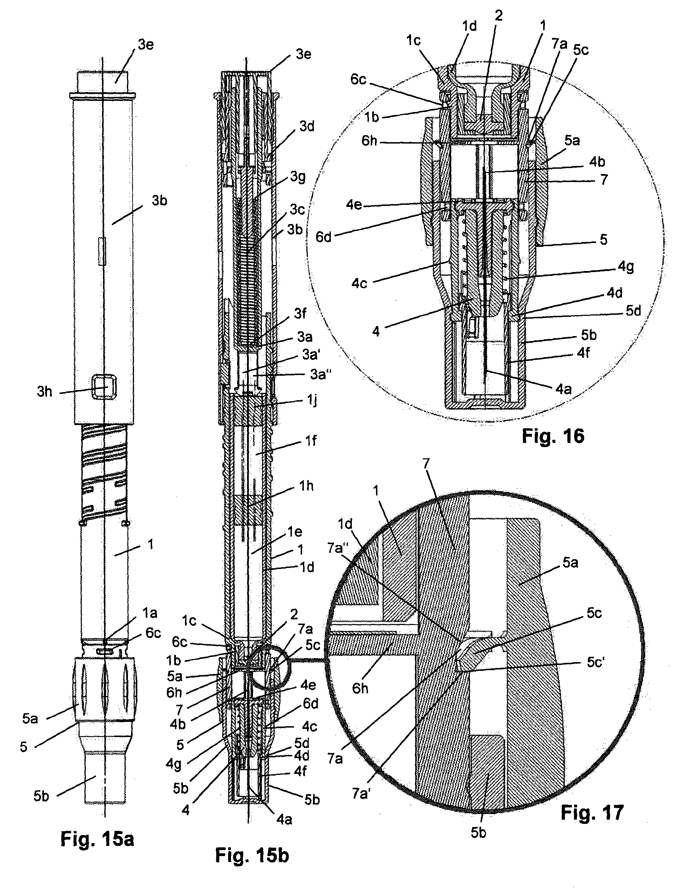

Die Figuren 15a und 15b zeigen eine Verabreichungsvorrichtung einer zweiten Ausführungsform in einer Ausgangsposition in Aussenansicht und im Längsschnitt. Die Verabreichungsvorrichtung unterscheidet sich von der Verabreichungsvorrichtung des ersten Ausführungsbeispiels im Wesentlichen nur in Bezug auf die Ausgestaltung der Führungshülse (7), in Bezug auf das Zusammenwirken der Führungshülse (7) mit dem Verriegelungselements (5c) und in Bezug auf das Zusammenwirken des Verriegelungselements (5c) mit dem Karpulenhalters (1). In Figur 16 ist eine vergrösserte Detailansicht im Längsschnitt der auf der Verabreichungsvorrichtung aufgesetzten Injektionsnadeleinheit (4) aus Figur 15b dargestellt. Wobei Figur 17 eine vergrösserte Detailansicht im Längsschnitt des Eingriffs zwischen dem Verriegelungselement (5c) der Schutzhülse (5) und einer Ausnehmung (7a) der Führungshülse (7) in der Ausgangsposition der auf der Verabreichungsvorrichtung aufgesetzten Injektionsnadeleinheit (4) aus Figur 15b zeigt. Die Führungshülse (7) kann über eine drehfeste Rippenverbindung mit der Schutzhülse (5) verbunden sein. Alternativ kann die Ausnehmung (7a) der Führungshülse (7) und das Verriegelungselement (5c) derart ausgebildet sein, dass die Führungshülse (7) mit der Schutzhülse (5) zumindest in der Ausgangsposition drehfest verbunden ist. Die Ausnehmung (7a) in der Führungshülse (7) weist eine steile (7a') und eine flache Kante (7a") auf. Das Verriegelungselement (5c) ist derart ausgebildet, dass es in der Ausgangsposition mit der Anschlagfläche (5c') gegen die steile Kante (7a') der Führungshülse (7) stösst, um zu verhindern, dass die Schutzhülse (5) mit der darin aufgenommenen Injektionsnadeleinheit (4) in die distale Richtung relativ zu der Führungshülse (7) bewegbar ist, insbesondere von der Injektionsnadeleinheit (4) abgezogen werden kann. In der Ausgangsposition wird die Schutzhülse (5) relativ zu der Führungshülse (7) über eine Nut-/Nockenverbindung axial festgehalten, insbesondere in die proximale Richtung axial festgehalten. Der Benutzer kann eine Kraft in die proximale Richtung, welche die Haltekraft der Nut-/Nockenverbindung übersteigt, gegen die Schutzhülse (5) aufbringen. Die Nut-/Nockenverbindung löst sich und die mit der Injektionsnadeleinheit (4) aufgenommene Schutzhülse (5) kann relativ zu dem Karpulenhalter (1) in die proximale Richtung bewegt werden, bis die Injektionsnadeleinheit (4) mit der Anschlagnocke oder dem Anschlagring (4e) gegen die distale Seite der Gegenanschlagebene (6h) der Führungshülse (6), welche auf dem distalen Ende des Karpulenhalters (1) aufliegt, stösst. Dabei gleitet das verformbar, insbesondere elastisch spannbar ausgebildete Verriegelungselement (5c) relativ zu der Führungshülse (7) über die flache Kante (7a") der Führungshülse (7) aus der Ausnehmung (7a) auf die Mantelaussenfläche der Führungshülse (7) heraus. Das Verriegelungselement (5c) wird dabei noch mehr gespannt. Dabei ist die Schutzhülse (5) und die Injektionsnadeleinheit (4) in Gleitkontakt mit der Führungshülse. Die Schutzhülse (5) mit der darin aufgenommenen Injektionsnadeleinheit (4) bewegt sich relativ zu der Führungshülse (7) in die proximale Richtung. Bei der Bewegung in die proximale Richtung durchdringt die Injektionsnadel der Injektionsnadeleinheit (4) die Membran der Karpule (1d). Das Verriegelungselement (5c) gleitet über das proximale Ende der Führungshülse (7) und kann sich zwischen der von der Schulter (1c) des Karpulenhalters (1) und dem proximalen Ende der Führungshülse (7) gebildeten Aussparung entspannen, insbesondere elastisch entspannen.

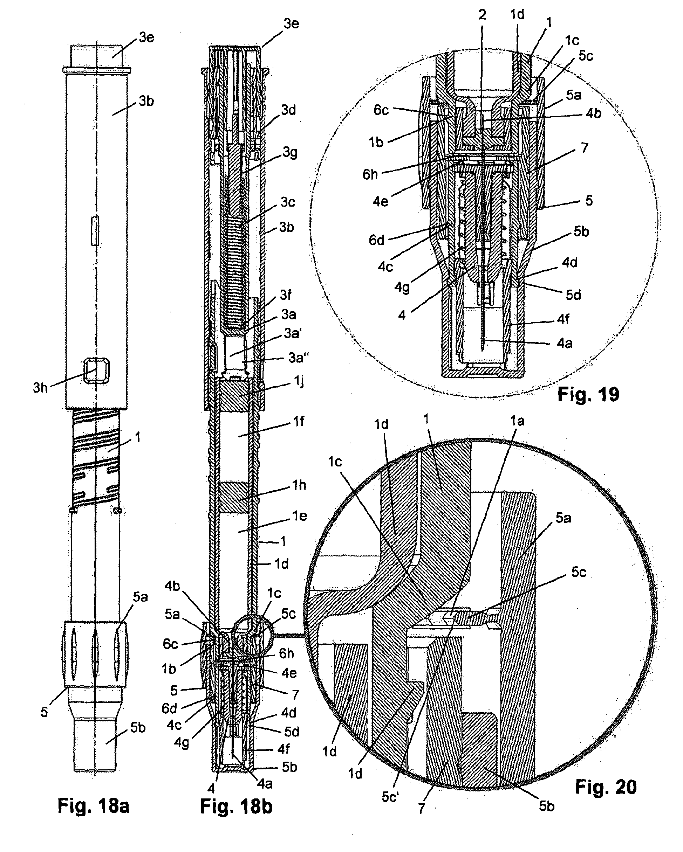

Die Figuren 18a und 18b zeigen die Verabreichungsvorrichtung der zweiten Ausführungsform in einer Abmischposition in Aussenansicht und im Längsschnitt. In Figur 19 ist eine vergrösserte Detailansicht im Längsschnitt der auf der Verabreichungsvorrichtung aufgesetzten Injektionsnadeleinheit (4) aus Figur 18b gezeigt. Figur 20 stellt eine vergrösserte Detailansicht im Längsschnitt des Eingriffs zwischen dem Verriegelungselement (5c) der Schutzhülse (5) und dem an dem Karpulenhalter angebrachten Vorsprung (1a), insbesondere Nocke (1 a) in der Abmischposition der auf der Verabreichungsvorrichtung aufgesetzten Injektionsnadeleinheit aus Figur 18b dar. Bei einer relativen Drehung der Schutzhülse (5) zu dem Karpulenhalter (1d), um den Wirkstoff mit der Lösungsflüssigkeit abzumischen, kann das entspannte, insbesondere elastisch entspannte Verriegelungselement (5c) der Schutzhülse (5) gegen einen an dem Karpulenhalter (1) vorgesehenen Vorsprung (1a), insbesondere Nocke (1a) stossen. Über die drehfeste Verbindung zwischen der Schutzhülse (5), insbesondere Verriegelungselement (5c) und dem Karpulenhalter (1), insbesondere Nocke (1a) kann der Karpulenhalter (1) im Gewindeeingriff mit dem Gehäuse (3b) in das Gehäuse (3b) geführt werden. Wenn der Abmischvorgang beendet ist, kann die Schutzhülse (5) von der Injektionsnadeleinheit (4) abgezogen werden, indem die Schutzhülse (5) relativ zu dem Karpulenhalter (1) in die distale Richtung bewegt wird. Das Verriegelungselement (5c) kann aufgrund seiner Verformbarkeit, insbesondere elastische Spannbarkeit über das proximale Ende des Karpulenhalters (1) gespannt werden, indem das Verriegelungselement (5c) über das proximale Ende des Karpulenhalters (1) relativ zu dem Karpulenhalter (1) gleitet. Das Verriegelungselement (5c) gleitet auf der Mantelaussenfläche der Führungshülse (7) relativ zu der Führungshülse (7) über die Aussparung der Führungshülse (7). Die Schutzhülse (5) kann somit von der Injektionsnadeleinheit (4) abgezogen werden.

### Bezugszeichen:

1 Karpulenhalter
1a Nocke
1b Keil
1c Schulter
1d Karpule
1e erste Kammer
1f zweite Kammer
1g Bypass
1h erster Stopfen
1j zweiter Stopfen
2 Membran
3a Kolbenstange
3a', 3a" Haltearme
3b Gehäuse
3c Feder
3d Blockierring
3e Auslöseknopf
3f Hülsenboden
3g gehäusefestes Element
3h Anzeigevorrichtung
4 Injektionsnadeleinheit
4a distaler Nadelabschnitt
4b proximaler Nadelabschnitt
4c Vorsprung
4d Nocke oder Ring
4e Anschlagnocke oder Anschlagring
4f Schutzschild
4g Feder
5 Schutzhülse
5a Griffteil
5b Schutzteil
5c Verriegelungselement
5c' Anschlagfläche
5d Ausnehmung oder Ringnut
6 Führungshülse
6a Schnapparm
6b Kulissenführung
6b' steile Stirnkante
6b" Rampe
6c Ausnehmung
6d Ausnehmung
6e Längssteg
6f Anschlagfläche
6g Anschlagfläche
6h Gegenanschlagebene
6j Rampe
7 Führungshülse
7a Ausnehmung
7a' steile Kante
7a" flache Kante

## Patentansprüche

1. Verabreichungsvorrichtung zur Verabreichung eines fluiden Wirkstoffs aus einem Behälter (1, 1d) mit
a) einem Behälter (1, 1d) mit wenigstens einer ersten Kammer (1e) mit einem Wirkstoff und einer zweiten Kammer (1f) mit einer Lösungsflüssigkeit für den Wirkstoff,
b) wobei der Behälter (1, 1d) zum Verschluss an einem Ende eine Membran (2) aufweist,
c) einer Abmischvorrichtung zum Abmischen des Wirkstoffs mit der Lösungsflüssigkeit für den Wirkstoff,
d) einer Injektionsnadeleinheit (4), die einen von dem Behälter (1, 1d) abgewandten distalen Nadelabschnitt (4a) zum Einstechen in die Haut und einen proximalen Nadelabschnitt (4b) aufweist, der in einer Ausgangsposition der Verabreichungsvorrichtung der Membran (2) zugewandt gegenüber liegt und der in einer Abmischposition durch die Membran (2) in den Behälter (1, 1d) gestochen ist, wobei
e) die Verabreichungsvorrichtung ferner eine Schutzhülse (5) aufweist, welche um die Injektionsnadeleinheit (4) koaxial angeordnet werden kann und welche in der Ausgangsposition relativ zu dem Behälter (1, 1d) mit der von der Schutzhülse (5) aufgenommenen Injektionsnadeleinheit (4) in die proximale Richtung, insbesondere nur in die proximale Richtung bewegbar ist, wobei
f) die Schutzhülle (5) in der Abmischposition drehfest mit dem Behälter (1, 1d) verbindbar ist und relativ zu der Abmischvorrichtung gedreht werden kann, um den Wirkstoff mit der Lösungsflüssigkeit für den Wirkstoff abzumischen,
**dadurch gekennzeichnet**,
g) dass die Verabreichungsvorrichtung ferner eine Führungshülse (6; 7) aufweist, wobei die Führungshülse (6; 7) radial zwischen der Injektionsnadeleinheit (4) und der Schutzhülse (5) angeordnet und an einem proximalen Ende mit dem Behälter (1, 1d) axial fest verbunden ist und, dass die Injektionsnadeleinheit (4) in der Führungshülse (6; 7) axial bewegbar aufgenommen ist und, dass die Schutzhülse (5) ein spannbares Verriegelungselement (5c) aufweist, welches in der Ausgangsposition derart mit der Führungshülse (6; 7) zusammenwirkt, dass die Schutzhülse (5) mit der darin aufgenommenen Injektionsnadeleinheit (4) relativ zu dem Behälter (1, 1d) in die proximale Richtung, insbesondere nur in die proximale Richtung bewegbar ist.

2. Verabreichungsvorrichtung nach Anspruch 1, **dadurch gekennzeichnet, dass** das Verriegelungselement (5c) der Schutzhülse (5) in der Abmischposition derart mit der Führungshülse (6; 7) zusammenwirkt, dass die Schutzhülse (5) relativ zu dem Behälter (1, 1d) in die distale Richtung bewegbar ist.

3. Verabreichungsvorrichtung nach Anspruch 1 oder 2, **dadurch gekennzeichnet, dass** die Schutzhülse (5) in der Ausgangsposition relativ zu dem Behälter (1, 1d) drehbar ist

4. Verabreichungsvorrichtung nach einem der Ansprüche 1 bis 3, **dadurch gekennzeichnet, dass** die Führungshülse (6; 7) in der Ausgangsposition oder in der Abmischposition relative zu dem Behälter (1, 1d) drehbar ist.

5. Verabreichungsvorrichtung nach einem der Ansprüche 1 bis 4, **dadurch gekennzeichnet, dass** die Schutzhülse (5) in der Ausgangsposition oder in der Abmischposition relativ zu der Führungshülse (6; 7) drehfest ist.

6. Verabreichungsvorrichtung nach einem der Ansprüche 1 bis 5, **dadurch gekennzeichnet, dass** die drehfeste Verbindung zwischen der Schutzhülse (5) und dem Behälter (1, 1d) in der Abmischposition durch einen Eingriff zwischen dem Behälter (5) und einem Schnapparm (6a), der an der Führungshülse (1) (6) angeordnet ist, herstellbar ist.

7. Verabreichungsvorrichtung nach Anspruch 6, **dadurch gekennzeichnet, dass** der Schnapparm (6a) In der Abmischposition radial nach innen spannbar ist.

8. Verabreichungsvorrichtung nach Anspruch 6 oder 7, **dadurch gekennzeichnet, dass** der Schnapparm (6a) in der Abmischposition gegen einen an dem Behälter (1, 1d) angebrachten Vorsprung (1a) angreift und den Eingriff zwischen dem Behälter (1) und dem Schnapparm (6a) bildet.

9. Verabreichungsvorrichtung nach einem der Ansprüche 1 bis 8, **dadurch gekennzeichnet, dass** die Führungshülse (6) eine Kulissenführung (6b) aufweist, wobei das Verriegelungselement (5c) entlang der Kulissenführung (6b) geführt werden kann.

10. Verabreichungsvorrichtung nach einem der Ansprüche 1 bis 5, **dadurch gekennzeichnet, dass** die drehfeste Verbindung zwischen der Schutzhülse (5) und dem Behälter (1, 1d) in der Abmischposition durch einen Eingriff zwischen dem Behälter (1, 1d) und einem Teil der Schutzhülse (5) oder dem Verriegelungselement (5c) der Schutzhülse (5) herstellbar ist.

11. Verabreichungsvorrichtung nach Anspruch 10, **dadurch gekennzeichnet, dass** der Behälter (1,1d) und das Teil der Schutzhülse (5) oder das Verriegelungselement (5c) der Schutzhülse (5) einen form- oder kraftschlüssigen Eingriff bilden.

12. Verabreichungsvorrichtung nach Anspruch 11, **dadurch gekennzeichnet, dass** der Eingriff eine Rippenverbindung, Nut/Keilverbindung, Stift-/Bohrungsverbindung, Anschlagverbindung oder Schnappverbindung ist.
(1) (1, 1d)

## Claims

1. Delivery device for the administration of a fluid active ingredient from a container (1, 1d) with
a) a container (1, 1d) with at least one first chamber (1e) with an active ingredient and a second chamber (If) with a solution liquid for the active ingredient,
b) wherein the container (1,1d) comprises a membrane (2) at one end for the closure,
c) a mixing device for the mixing of the active ingredient with the solution liquid for the active ingredient,
d) an injection needle unit (4), which comprises a distal needle portion (4a) facing away from the container (1,1d) for injection into the skin and a proximal needle portion (4b), which, in a starting position of the delivery device, is disposed facing opposite to the membrane (2), which, in a mixing position, pierces through the membrane (2) into the container (1, 1d), wherein
e) the delivery device further comprises a protective sleeve (5) which can be arranged coaxially around the injection-needle unit (4) and which, in the starting position, with the injection-needle unit (4) retained by the protective sleeve (5), is displaceable relative to the container (1,1d) in the proximal direction, especially only in the proximal direction, wherein,
f) in the mixing position, the protective sleeve (5) is connectable in a rotationally rigid manner to the container (1,1d) and can be rotated relative to the mixing device, in order to mix the active ingredient with the solution liquid for the active ingredient,
**characterised in that**,
g) The delivery device further comprises a guide sleeve (6; 7), wherein the guide sleeve (6; 7) is arranged radially between the injection-needle unit (4) and the protective sleeve (5) and is connected at a proximal end to the container (1, 1 d) in an axially rigid manner and, that the injection-needle unit (4) is retained in the guide sleeve (6; 7) in an axially displaceable manner and, that the protective sleeve (5) comprises a tensible locking element (5c), which, in the starting position, cooperates with the guide sleeve (6; 7) in such a manner that the protective sleeve (5) with the injection-needle unit (4) retained therein is displaceable relative to the container (1, 1d) in the proximal direction, especially only in the proximal direction.

2. Delivery device according to claim 1, **characterised in that**, in the mixing position, the locking element (5c) of the protective sleeve (5) cooperates with the guide sleeve (6; 7) in such a manner that the protective sleeve (5) is displaceable relative to the container (1, 1d) in the distal direction.

3. Delivery device according to claim 1 or 2, **characterised in that**, in the starting position, the protective sleeve (5) is rotatable relative to the container (1, 1d).

4. Delivery device according to one of claims 1 to 3, **characterised in that**, in the starting position or in the mixing position, the guide sleeve (6; 7) is rotatable relative to the container (1, 1d).

5. Delivery device according to one of claims 1 to 4, **characterised in that**, in the starting position or in the mixing position, the protective sleeve (5) is rotationally rigid relative to the guide sleeve (6; 7).

6. Delivery device according to one of claims 1 to 5, **characterised in that** the rotationally rigid connection between the protective sleeve (5) and the container (1, 1d) can be established in the mixing position by an engagement between the container (1, 1d) and a snap arm (6a) which is arranged on the guide sleeve (6).

7. Delivery device according to claim 6, **characterised in that**, in the mixing position, the snap arm (6a) is tensible radially inwards.

8. Delivery device according to claim 6 or 7, **characterised in that**, in the mixing position, the snap arm (6a) engages against a projection (1a) fitted to the container (1, 1d) and forms the engagement between the container (1) and the snap arm (6a).

9. Delivery device according to one of claims 1 to 8, **characterised in that** the guide sleeve (6) comprises a sliding-block guide (6b), wherein the locking element (5c) can be guided along the sliding-block guide (6b).

10. Delivery device according to one of claims 1 to 5, **characterised in that** the rotationally rigid connection between the protective sleeve (5) and the container (1, 1d) can be established in the mixing position by an engagement between the container (1, 1d) and a part of the protective sleeve (5) or the locking element (5c) of the protective sleeve (5).

11. Delivery device according to claim 10, **characterised in that** the container (1, 1d) and the part of the protective sleeve (5) or the locking element (5c) of the protective sleeve (5) form a form-fit or force-fit engagement.

12. Delivery device according to claim 11, **characterised in that** the engagement is a rib connection, wedge/groove connection, pin/hole connection, notch connection or snap connection.

## Revendications

1. Dispositif d'administration pour l'administration d'une substance active fluide provenant d'un récipient (1, 1d) avec
a) un récipient (1, 1d) avec au moins une première chambre (1e) avec une substance active et une deuxième chambre (1f) avec un liquide de mise en solution pour la substance active,
b) le récipient (1, 1d) comprend, pour la fermeture, au niveau d'une extrémité, une membrane (2),
c) un dispositif de mélange pour le mélange de la substance active avec le liquide de mise en solution pour la substance active,
d) une unité d'aiguille d'injection (4) qui comprend une portion d'aiguille distale (4a) opposée au récipient (1, 1d), à enfoncer dans la peau et une portion d'aiguille proximale (4b) qui fait face, dans une position initiale du dispositif d'administration, à la membrane (2), et qui, dans une position de mélange, est enfoncée à travers la membrane (2), dans le récipient (1, 1d),
e) le dispositif d'administration comprenant en outre un manchon de protection (5) qui peut être disposé de manière coaxiale autour de l'unité d'aiguille d'injection (4) et qui est mobile, dans la position initiale, par rapport au récipient (1, 1d), avec l'unité d'aiguille d'injection (4) logée par le manchon de protection (5), dans la direction proximale, plus particulièrement uniquement dans la direction proximale,
f) le manchon de protection (5) pouvant être relié, dans la position de mélange, de manière solidaire en rotation avec le récipient (1, 1d) et pouvant être tourné par rapport au dispositif de mélange afin de mélanger la substance active avec le liquide de mise en solution pour la substance active,
**caractérisé en ce que**
g) le dispositif d'administration comprend en outre un manchon d'introduction (6 ; 7), le manchon de guidage (6 ; 7) étant disposé radialement entre l'unité d'aiguille d'injection (4) et le manchon de protection (5) et étant relié de manière fixe axialement, au niveau d'une extrémité proximale, avec le récipient (1, 1d) et **en ce que** l'unité d'aiguille d'injection (4) est logée de manière mobile axialement dans le manchon de guidage (6 ; 7) et **en ce que** le manchon de protection (5) comprend un élément de verrouillage (5c) qui peut être serré, lequel interagit dans la position initiale avec le manchon de guidage (6 ; 7) de telle sorte que le manchon de protection (5) est mobile, avec l'unité d'aiguille d'injection (4) logée à l'intérieur, par rapport au récipient (1, 1d), dans la direction proximale, plus particulièrement uniquement dans la direction proximale.

2. Dispositif d'administration selon la revendication 1, **caractérisé en ce que** l'élément de verrouillage (5c) du manchon de protection (5) interagit, dans la position de mélange, avec le manchon de guidage (6 ; 7), de façon à ce que le manchon de protection (5) soit mobile par rapport au récipient (1, 1d) dans la direction distale.

3. Dispositif d'administration selon la revendication 1 ou 2, **caractérisé en ce que** le manchon de protection (5) est rotatif dans la position initiale par rapport au récipient (1, 1d).

4. Dispositif d'administration selon l'une des revendications 1 à 3, **caractérisé en ce que** le manchon de guidage (6 ; 7) est rotatif dans la position initiale ou dans la position de mélange par rapport au récipient (1, 1d).

5. Dispositif d'administration selon l'une des revendications 1 à 4, **caractérisé en ce que** le manchon de protection (5) est solidaire en rotation dans la position initiale ou dans la position de mélange par rapport au manchon de guidage (6 ; 7).

6. Dispositif d'administration selon l'une des revendications 1 à 5, **caractérisé en ce que** la liaison solidaire en rotation entre le manchon de protection (5) et le récipient (1, 1d) peut être établie, dans la position de mélange, par un emboîtement entre le récipient (1, 1d) et un bras d'encliquetage (6a), qui est disposé sur le manchon de guidage (6).

7. Dispositif d'administration selon la revendication 6, **caractérisé en ce que** le bras d'encliquetage (6a) peut être serré radialement vers l'intérieur dans la position de mélange.

8. Dispositif d'administration selon la revendication 6 ou 7, **caractérisé en ce que** le bras d'encliquetage (6a) s'emboîte, dans la position de mélange, contre une saillie (1a) réalisée sur le récipient (1, 1d) et forme l'emboîtement entre le récipient (1) et le bras d'encliquetage (6a).

9. Dispositif d'administration selon l'une des revendications 1 à 8, **caractérisé en ce que** le manchon de guidage (6) comprend un guidage coulissant (6b), l'élément de verrouillage (5c) pouvant être guidé le long du guidage coulissant (6b).

10. Dispositif d'administration selon l'une des revendications 1 à 5, **caractérisé en ce que** la liaison solidaire en rotation entre le manchon de protection (5) et le récipient (1, 1d) peut être établie, dans la position de mélange, par un emboîtement entre le récipient (1, 1d) et une partie du manchon de protection (5) ou l'élément de verrouillage (5c) du manchon de protection (5).

11. Dispositif d'administration selon la revendication 10, **caractérisé en ce que** le récipient (1, 1d) et la partie du manchon de protection (5) ou l'élément de verrouillage (5c) du manchon de protection (5) forment un emboîtement par complémentarité de forme ou par friction.

12. Dispositif d'administration selon la revendication 11, **caractérisé en ce que** l'emboîtement est une liaison par nervures, une liaison rainure/clavette, une liaison goupille/alésage, une liaison à butée ou une liaison par encliquetage.
